**Europäisches Patentamt**

**European Patent Office**

**Office européen des brevets**

(11) Veröffentlichungsnummer: **0 043 149**
**A1**

(12) # EUROPÄISCHE PATENTANMELDUNG

(21) Anmeldenummer: **81200608.8**

(22) Anmeldetag: **03.06.81**

(51) Int. Cl.³: **A 61 B 5/10**
**A 61 C 19/04**

(30) Priorität: **24.06.80 CH 4846/80**

(43) Veröffentlichungstag der Anmeldung:
**06.01.82 Patentblatt 82/1**

(84) Benannte Vertragsstaaten:
**AT BE DE FR GB IT LU NL SE**

(71) Anmelder: **Balogh, József**
**Diebold Schillingstrasse 24**
**CH-6400 Luzern (Kanton Luzern)(CH)**

(72) Erfinder: **Balogh, József**
**Diebold Schillingstrasse 24**
**CH-6400 Luzern (Kanton Luzern)(CH)**

(74) Vertreter: **Bovard, Fritz Albert et al,**
**Bovard & Cie Patentanwälte VSP Optingenstrasse 16**
**CH-3000 Bern 25(CH)**

(54) **Verfahren und Vorrichtung zum Registrieren der Unterkieferbewegungen eines Patienten.**

(57) Die Bewegungen des Unterkiefers eines Patienten beim Kauen oder Sprechen werden mit Ultraschallwellen gemessen. Die so erhaltenen Messwerte werden in einem Elektronenrechner ausgewertet und gespeichert. Die Ultraschallwellen werden durch Sendeköpfe (2) ausgestrahlt, die an einem Ende einer Stange (1) elektrisch isoliert angeordnet sind. Das andere Ende der Stange (1) ist an einer an den Unterkiefer des Patienten aufzusetzenden Mittel befestigt, so dass die Bewegungen des Unterkiefers auf die Stange (1) übertragen werden. Das mit den Sendeköpfen (2) versehene Ende der Stange (1) ist in ein Gehäuse (7) mit Ultraschallempfängerköpfen (8) einführbar. Die von den Sendeköpfen (2) ausgestrahlten Ultraschallsignale werden von Empfängerköpfen (8) empfangen und weiter zu einem Elektrodenenrechner in einem anderen Gehäuse (3) geleitet. Im Elektronenrechner werden die Messwerte ausgewertet und gespeichert. In diesem zahnmedizinischen Ulatraschall-Messgerät werden einzelne Unterkieferbewegungen des Patienten genau gemessen, sofort ausgewertet und kontinuierlich angezeigt sowie gespeichert.

EP 0 043 149 A1

Fig. 2

Fig. 1

**Croydon Printing Company Ltd.**

## Verfahren und Vorrichtung zum Registrieren der Unterkieferbewegungen eines Patienten

Die Erfindung geht aus von einem Verfahren zum Registrieren der Unterkieferbewegungen eines Patienten beim Kauen oder Sprechen sowie einer Vorrichtung zum Ausführen des Verfahrens, mit einem ersten Gehäuse, in welchem ein Elektronenrechner, eine Digital-Anzeigeeinheit, ein Matrixdrucker und eine Bedienungsapparatur untergebracht sind.

Für die Herstellung von Gebissen, Brücken, Prothesen und für andere zahnmedizinische Untersuchungen sind für einen Arzt die Bewegungen des unteren Kiefers gegenüber dem oberen Kiefer des Patienten von grosser Bedeutung. So zum Beispiel gewährleisten die richtige Anordnung, Abstand und Eingriff der hergestellten Prothesen usw., die den wirklichen Bedingungen beim Kauen oder Sprechen am genauesten entsprechen sollen, einen angenehmen, ungestörten Gebrauch durch den Patienten.

Der Unterkiefer führt während des Kauens oder Sprechens eine komplizierte Bewegung aus, die sehr individuduell ist. Er führt eine kauende, mahlende Bewegung nach vorn und im Halbkreis aus; er adaptiert, artikuliert und okkludiert. Diese individuellen Bewegungen des Unterkiefers sind von der Gestaltung der Kugelpfanne im Schädel des Patienten abhängig, in welcher die Gelenke des Unterkiefers eingesetzt sind. Auch die Neigung der die Gelenke und die Mundöffnung eines Patienten durchlaufenden Ebene ist sehr unterschiedlich.

Bisher wurden die Unterkieferbewegungen eines Patienten mittels verschiedener mechanischer Messgeräte gemessen, womit aber keine präzisen Resultate erreicht werden konnten. Dies führte dazu, dass die auf Grund solcher Messungen hergestellten Einsätze oder Prothesen öfters gerichtet werden mussten; durch die mechanische Messung kann nämlich die individuelle Unterkieferbewegung nie richtig erfasst werden.

Der im Anspruch 1 angegebenen Erfindung liegt die Aufgabe zugrunde, die Nachteile der mechanisch arbeitenden Messgeräte zu beseitigen und ein Verfahren zum Registrieren der Unterkieferbewegungen eines Patienten beim Kauen oder Sprechen vorzuschlagen und eine Vorrichtung zum Ausführen desselben zu schaffen, mittels welcher einzelne Unterkieferbewegungen des Patienten genau gemessen und sofort ausgewertet werden können.

Diese Aufgabe wird bei einem Verfahren nach dem Oberbegriff des Patentanspruches 1 dadurch gelöst, dass die Bewegungen des Unterkiefers mit Ultraschallwellen gemessen und die so erhaltenen Messwerte in einem Elektronenrechner ausgewertet und gespeichert werden.

Die erfindungsgemässe Vorrichtung zum Ausführen des Verfahrens ist gekennzeichnet durch eine mit dem Unterkiefer des Patienten in Verbindung bringbare und durch die indivuduellen Unterkieferbewegungen betätigbare Stange mit von ihr elektrisch isolierten Ultraschallsendeköpfen und ein zweites Gehäuse mit Ultraschallempfängerköpfen, in welches die Stange mit ihrem die Ultraschallsendeköpfe tragenden Ende einführbar ist.

Mit Vorteil werden die Messwerte der Sagital- und Scharnierbewegungen, der Lateralbewegungen und der Oeffnungs- und Schliessbewegungen des Unterkiefers in Grad angezeigt, wogegen die Messwerte der Bewegungen des Unterkiefers nach vorne in Millimetern angezeigt werden.

Die Erfindung wird nachstehend anhand der Zeichnungen beispielsweise näher erläutert. Es zeigen

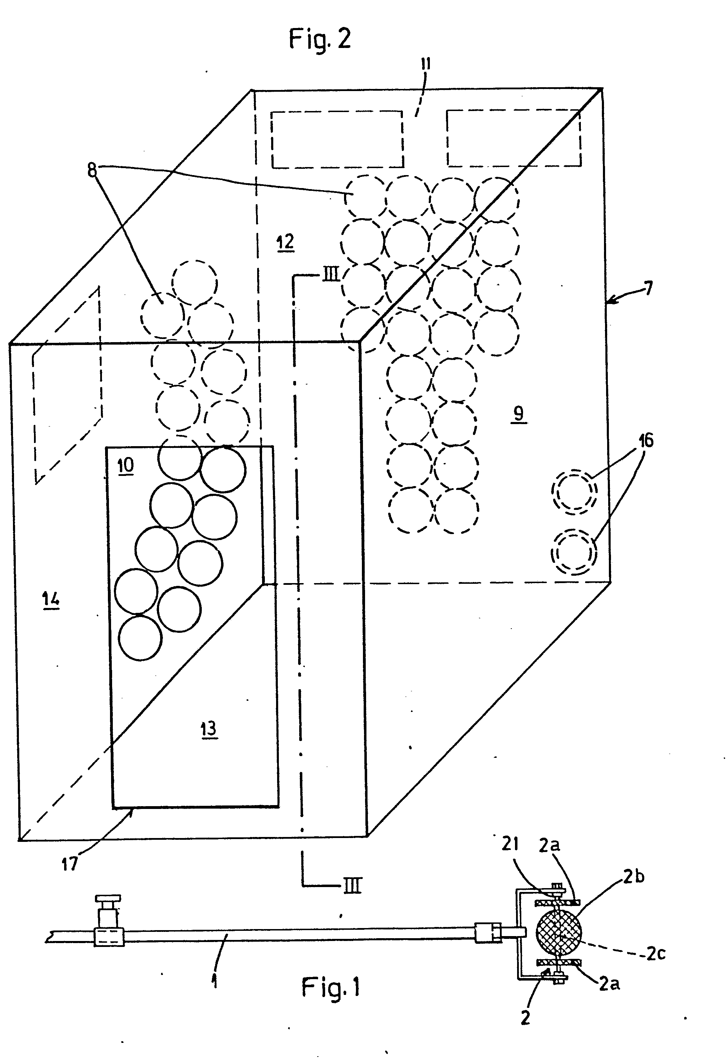

Fig. 1 eine Draufsicht auf eine mit dem Unterkiefer des Patienten in Verbindung bringbare Stange mit vier elektrisch isolierten Ultraschallsendeköpfen an einem ihrer Enden,

Fig. 2 eine schematische Darstellung eines Gehäuses mit an den Innenseiten seiner Wände mit Ausnahme der einen eine Oeffnung aufweisenden Wand und des Bodens montierten Ultraschallempfängerköpfen,

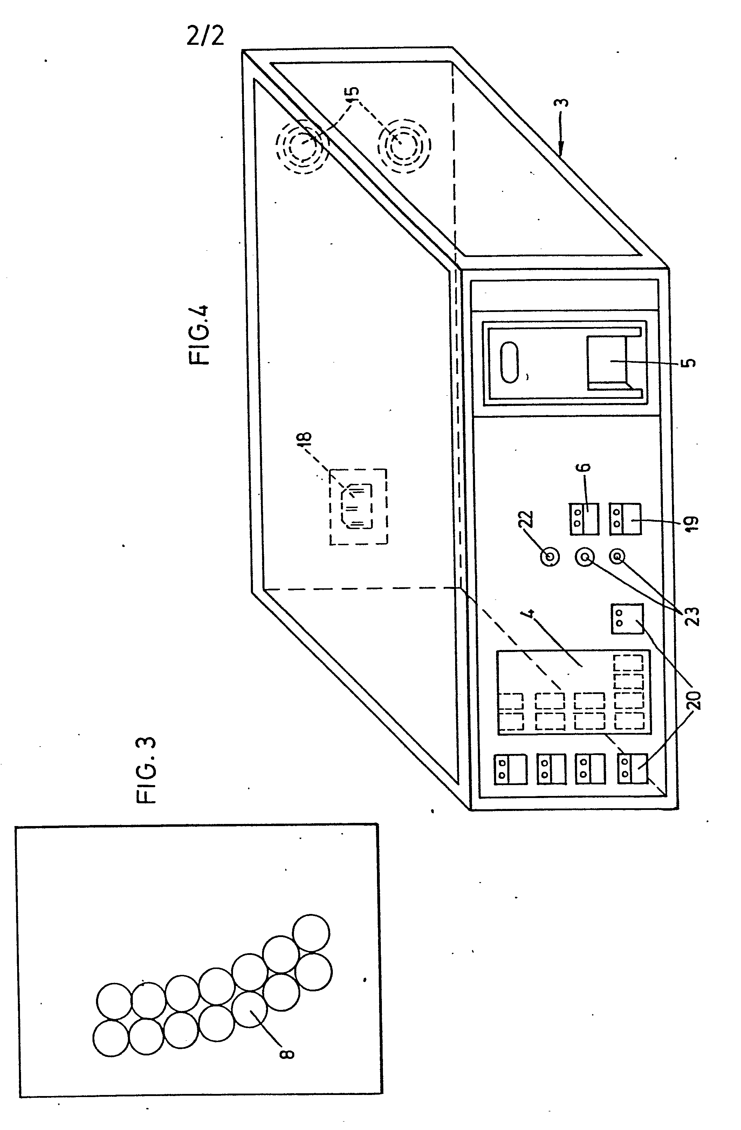

Fig. 3 einen Schnitt entlang der Linie III - III der Fig. 2 und

Fig. 4 eine schematische Ansicht eines weiteren Gehäuses mit einem Elektronenrechner, einer Digitalanzeigeeinheit, einem Matrixdrucker und einer Bedienungsapparatur.

Während des Kauens oder Sprechens macht der Unterkiefer eines Patienten komplizierte Bewegungen. Es sind dies

a) Sagital- und Scharnierbewegungen oder besser gesagt Bewegungen des Unterkiefers, die bezogen auf die Sagitalebene senkrecht verlaufen. Bei solchen Bewegungen kann die Schwenkachse, um welche der Unterkiefer bewegt wird, stationär innerhalb der Gelenke bleiben. In einem solchen Fall bewegt sich der Unterkiefer auf einer Bahn, die nicht länger als 1,8 bis 2,5 cm ist. Wenn die Schwenkachse, um welche der Unterkiefer bewegt wird, weiter nach unten versetzt wird, verschieben sich auch die Unterkiefergelenke nach vorne und nach unten.

b) Lateralbewegungen des Unterkiefers nach links und rechts; diese Bewegungen werden senkrecht zur Horizontalebene projiziert. Auch bei diesen Bewegungen verschieben sich die Unterkiefergelenke. Die Bewegungsbahn ist hier 1,5 bis 2 cm lang.

c) Oeffnungs- und Schliessbewegungen (Bisswinkel)
des Unterkiefers werden auf eine zur der Frankfurter-
Horizontalebene senkrecht verlaufende Linie und eine
die Schneidezähne des Oberkiefers und Schneidezähne des
im geöffneten Zustand befindlichen Unterkiefers durchlaufende Ebene bezogen.

d) Bewegungen des Unterkiefers nach vorne werden
von der Stirnfläche der Schneidezähne des Oberkiefers
gemessen. Die Bewegungsbahn beträgt etwa 1 cm.

Um die Unterkieferbewegungen eines Patienten beim
Kauen oder Sprechen registrieren zu können, werden diese
Bewegungen mit Ultraschallwellen gemessen und die so
erhaltenen Messwerte in einem Elektronenrechner ausgewertet und gespeichert.

Die Vorrichtung zum Ausführen dieser Verfahrensschritte weist ein erstes Gehäuse 3, ein zweites Gehäuse 7 und eine Stange mit elektrisch isolierten Ultraschallsendeköpfen 2 auf.

Im ersten Gehäuse ist ein durch einen Mikroprozessor
gesteuerter, nicht dargestellter Elektronenrechner eingebaut, in welchem die durch Ultraschallwellen gemessenen
Werte verarbeitet werden. Die Datenverarbeitung erfolgt
auf 16 Bits-Basis. In dem ersten Gehäuse 3 ist weiter
eine Digital-Anzeigeeinheit 4, in welcher fünf Momentanwerte kontinuierlich angezeigt werden, ein Matrixdrucker 5 und eine Bedienungsapparatur mit Bedienungselementen 6, 9 und 20 untergebracht. Die Bedienungselemente umfassen eine Start/Stop Taste 6, eine Taste 19
zur Betätigung des Matrixdruckers 5 und fünf Tasten 20
zum Speichern der Messergebnisse. Alle Bedienungselemente sind als beschriftete Leuchtdrucktasten mit
Leuchtdioden ausgebildet. Mit 22 ist eine Eichtaste und
mit 23 sind Netzschalter bezeichnet.

Das erste Gehäuse 3, das an einen Netzstrom anschliessbar ist, ist über zwei Steckdosen 15 mit einem
zweiten Gehäuse 7 mit Steckdosen 16 elektrisch verbunden.

Das zweite Gehäuse 7 ist mit Ultraschallempfängerköpfen 8 versehen, die scheibenförmig ausgebildet sind. Sie sind an den Innenseiten der Gehäusewände 9 bis 12 mit Ausnahme der eine Oeffnung 17 aufweisenden Wand 14 und des Bodens 13 angeordnet. Die an den Innenseiten der gegenüberliegenden Seitenwände 9, 10 angeordneten Ultraschallempfängerköpfe 8 sind bogenförmig gruppiert.

Die Vorrichtung enthält weiter eine Stange 1, die an einem ihrer Enden vier scheibenförmige, von der Stange 1 elektrisch isolierte Ultraschallsendeköpfe 2 trägt. Die Ultraschallsendeköpfe 2 sind so angeordnet, dass zwei gegenüberliegende Scheiben 2a zueinander parallel, jedoch zu den übrigen zwei Scheiben 2b, 2c senkrecht verlaufen. Die vier Ultraschallsendeköpfe 2 sind an einer in Kugellagern laufenden Welle 21 befestigt und so ausbalanciert, dass die beiden gegenüberliegenden und zueinander parallel verlaufenden Sendeköpfe 2a zu den Seitenwänden 9 und 10 und die übrigen zwei zueinander senkrecht angeordneten Sendeköpfe 2b, 2c zu der Rückwand 11 und der Decke 12 immer parallel verlaufen.

Die Stange 1 ist mit ihrem die Ultraschallsendeköpfe 2 tragenden Ende durch die Oeffnung 17 in den Innenraum des zweiten Gehäuses 7 einführbar. Die bogenförmige Gruppierung der Ultraschallempfängerköpfe 8 auf den Innenseiten der gegenüberliegenden Seitenwände 9, 10 entspricht im Grunde den Bewegungsbahnen der zwei zueinander parallel verlaufenden Ultraschallsendeköpfe 2a. Die geradlinige Gruppierung der Ultraschallempfängerköpfe 8 auf den Innenseiten der Rückwand 11 und der Decke 12 entspricht den Bewegungsbahnen der zwei übrigen Ultraschallsendeköpfe 2b, 2c.. Die Stange 1 ist mit ihrem anderen Ende mit einem an den Unterkiefer des Patienten aufzusetzenden, nicht dargestellten Mittel verbunden, so dass die Bewegungen des Unterkiefers auf sie übertragen werden.

Die Ultraschallsendeköpfe 2 sind mit dem zweiten Gehäuse 7 elektrisch verbunden. Der Grundtakt des Elektrostroms in der Vorrichtung ist 10 MHz.

Die Sendeköpfe 2 strahlen Ultraschallwellen aus, die von Empfängerköpfen 8 empfangen werden. Die Entfernungsmessung der einzelnen Bewegungsbahnen des Unterkiefers, die auf die Stange 1 übertragen werden, erfolgt nach dem Ultraschallzeitmultiplex-Messverfahren-Prinzip. Die Ultraschallsignale werden als Messwerte zu dem nichtdargestellten Elektronenrechner im ersten Gehäuse 3 geleitet, wo sie ausgewertet und gespeichert werden. In der Digitalanzeigeeinheit 4 werden die Messwerte der Sagital- und Scharnierbewegungen, der Lateralbewegungen und der Oeffnungs- und Schliessbewegungen des Unterkiefers in Grad als Momentanwerte kontinuierlich angezeigt. Die Messwerte der Bewegungen des Unterkiefers nach vorne werden in Millimetern als Momentanwerte kontinuierlich angezeigt.

Die ausgewählten Momentanwerte werden nach der Betätigung der entsprechenden Bedienungselemente der Bedienungsapparatur gespeichert werden. Die gespeicherten Messwerte können in einem beliebigen Zeitpunkt mit Matrixdrucker 5 protokolliert werden. Die Messung ist jederzeit mit Hilfe der Stop/Start Taste 6 unterbrechbar bzw. fortsetzbar.

Mit Hilfe der oben beschriebenen, als zahnmedizinisches Ultraschall-Messgerät dienenden Vorrichtung können individuelle Unterkieferbewegungen des Patienten mit einer Genauigkeit von 0,3° bzw. 0,3 mm erfasst werden. Die Gehäuse 3 und 7 können auf einem drehbaren, höhenverstellbaren Tisch angeordnet sein, um das Messgerät je nach Wunsch einstellen zu können. Mit Hilfe dieses Messgerät können einzelne Phasen der individuellen und komplizierten dreidimensionalen Unterkieferbewegungsbahnen ununterbrochen registriert werden und die Messwerte abgelesen und gespeichert werden.

**0043149**

PATENTANSPRUECHE

1. Verfahren zum Registrieren der Unterkieferbewegungen eines Patienten beim Kauen oder Sprechen, dadurch gekennzeichnet, dass die Bewegungen des Unterkiefers mit Ultraschallwellen gemessen und die so erhaltenen Messwerte in einem Elektronenrechner ausgewertet und gespeichert werden.

2. Verfahren nach Patentanspruch 1, dadurch gekennzeichnet, dass die Messwerte der Sagital- und Scharnierbewegungen, der Lateralbewegungen und der Oeffnungs- und Schliessbewegungen des Unterkiefers in Grad angezeigt werden.

3. Verfahren nach Patentanspruch 1, dadurch gekennzeichnet, dass die Messwerte der Bewegungen des Unterkiefers nach vorne in Millimetern angezeigt werden.

4. Verfahren nach den Patentansprüchen 2 und 3, dadurch gekennzeichnet, dass die Messwerte aller Bewegungen des Unterkiefers als Momentanwerte kontinuierlich angezeigt werden.

5. Vorrichtung zum Ausführen des Verfahrens nach Patentanspruch 1, mit einem ersten Gehäuse (3), in welchem ein Elektronenrechner, eine Digital-Anzeigeeinheit (4), ein Matrixdrucker (5) und eine Bedienungsapparatur (6) untergebracht sind, gekennzeichnet durch eine mit dem Unterkiefer des Patienten in Verbindung bringbare und durch die individuellen Unterkieferbewegungen betätigbare Stange (1) mit von ihr elektrisch isolierten Ultraschallsendeköpfen (2) und ein zweites Gehäuse (7) mit Ultraschallempfängerköpfen (8), in welches die Stange (1) mit ihrem die Ulatraschallsendeköpfe (2) tragenden Ende einführbar ist.

6. Vorrichtung nach Patentanspruch 5, dadurch gekennzeichnet, dass die Stange (1) vier scheibenförmige Ultraschallsendeköpfe (2) trägt, deren die zwei gegenüberliegenden Scheiben (2a) zueinander parallel, jedoch zu den übrigen zwei Scheiben (2b, 2c) senkrecht verlaufen.

7. Vorrichtung nach Patentanspruch 5, dadurch gekennzeichnet, dass die Ultraschallempfängerköpfe (8) des zweiten Gehäuses (7) ebenfalls scheibenförmig ausgebildet und an den Innenseiten der Gehäusewände (9 bis 12) mit Ausnahme der eine Oeffnung (17) zum Einführen der Stange (1) aufweisenden Wand (14) und des Bodens (13) angeordnet sind.

8. Vorrichtung nach Patentanspruch 7, dadurch gekennzeichnet, dass die an den Innenseiten der gegenüberliegenden Seitenwände (9, 10) angeordneten Ultraschallempfängerköpfe (8) bogenförmig gruppiert sind.

9. Vorrichtung nach einem der Patentansprüche 5 bis 8, dadurch gekennzeichnet, dass die Ultraschallsendeköpfe (2) mit dem zweiten Gehäuse (7) und das zweite Gehäuse (7) mit dem ersten Gehäuse (3) elektrisch verbunden sind.

10. Vorrichtung nach Patentanspruch 9, dadurch gekennzeichnet, dass der Grundtakt des Elektrostromes 10 MHz beträgt.

1/2

0043149

Fig. 2

Fig. 1

FIG. 3

FIG.4

0043149

# 0043149

Nummer der Anmeldung

EP 81 20 0608

**Europäisches Patentamt**

## EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| A | US - A - 3 822 694 (G.E. MILLS) <br> * Zusammenfassung; Spalte 2, Zeilen 16-22; Spalte 2, Zeile 33 - Spalte 3, Zeile 25; Abbildung 1 * <br> -- | 1,5 |
| A | EP - A - 0 004 888 (SIEMENS AKTIEN-GESELLSCHAFT) <br> * Zusammenfassung; Abbildung 1 * <br> -- | 1,5 |
| A | DE - A - 2 415 309 (W. WAGNER) <br> * Seite 1, Zeilen 7-15; Seite 4, Zeilen 3-20; Abbildungen 2,4 * <br> -- | 1,5 |
| A | US - A - 3 699 856 (R.W. CHABOT et al.) <br> * Zusammenfassung; Abbildungen 1-3 * <br> ---- | 1,5 |

### KLASSIFIKATION DER ANMELDUNG (Int. Cl.³)

A 61 B  5/10
A 61 C  19/04

### RECHERCHIERTE SACHGEBIETE (Int. Cl.³)

A 61 B  5/10
        10/00
A 61 C  19/04

### KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patent-familie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 06-10-1981 | BEAVEN |

EPA form 1503.1  06.78